(19) Europäisches Patentamt
European Patent Office
Office européen des brevets

(11) Veröffentlichungsnummer : **0 161 485**
**B1**

(12) **EUROPÄISCHE PATENTSCHRIFT**

(45) Veröffentlichungstag der Patentschrift : **17.08.88**

(51) Int. Cl.⁴ : **C 07 C179/10**, C 11 D 3/39

(21) Anmeldenummer : **85104345.5**

(22) Anmeldetag : **10.04.85**

# (54) Verfahren zur Herstellung wasserunlöslicher Peroxycarbonsäuren.

(30) Priorität : **18.05.84 DE 3418450**

(43) Veröffentlichungstag der Anmeldung : **21.11.85 Patentblatt 85/47**

(45) Bekanntmachung des Hinweises auf die Patenterteilung : **17.08.88 Patentblatt 88/33**

(84) Benannte Vertragsstaaten : **AT BE CH DE FR GB IT LI LU NL SE**

(56) Entgegenhaltungen :
**EP-A- 0 023 545**
**EP-A- 0 127 782**
**US-A- 2 609 391**
**US-A- 4 119 660**

(73) Patentinhaber : **Degussa Aktiengesellschaft**
**Weissfrauenstrasse 9**
**D-6000 Frankfurt am Main 1 (DE)**

**Henkel Kommanditgesellschaft auf Aktien**
**Postfach 1100 Henkelstrasse 67**
**D-4000 Düsseldorf-Holthausen (DE)**

(72) Erfinder : **Dankowski, Manfred, Dr.**
**Stifterstrasse 14**
**D-8757 Karlstein (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 161 485 B1**

Jouve, 18, rue St-Denis, 75001 Paris, France

**Beschreibung**

Die Erfindung betrifft ein Verfahren zur Herstellung wasserunlöslicher Peroxycarbonsäuren.

Derartige Verbindungen werden nicht nur als Oxidationsmittel in der organischen Synthese eingesetzt sondern auch bei der Wäsche oder Bleiche von Textilien, da ihre Wirkung schon bei Temperaturen unterhalb von 80 °C eintritt.

Es ist bereits eine Reihe verschiedener Verfahren zur Herstellung von wasserunlöslichen Peroxycarbonsäuren veröffentlicht worden.

Die EP-A-2-0045290 betrifft ein Verfahren zur Herstellung von Peroxycarbonsäuren, bei dem die Ausgangscarbonsäuren in konzentrierter Schwefelsäure gelöst, und die durch Oxidation mit Wasserstoffperoxid gebildeten Persäuren kontinuierlich mit einem organischen Lösungsmittel extrahiert werden.

In der US-PS-4 119 660 wird ebenfalls ein Verfahren beschrieben, bei dem die Ausgangscarbonsäuren in großen Überschüssen von Schwefelsäure gelöst und anschließend zu Persäuren umgesetzt werden.

Die Schwefelsäure wirkt bei diesen Verfahren als Katalysator und Lösungsmittel für die Ausgangscarbonsäure.

Die katalytische Wirkung tritt aber auch schon bei geringeren Konzentrationen ein. So richtet sich die EP-A-00 23 545 auf ein Verfahren zur Herstellung aromatischer Peroxycarbonsäuren, wobei die feste aromatische Carbonsäure in ein Gemisch aus wässrigem Wasserstoffperoxid und Schwefelsäure eingetragen wird und die Reaktion in heterogener Phase stattfindet. Das Molverhältnis Carbonsäure zu Wasserstoffperoxid zu Schwefelsäure liegt zwischen 1 : 2 : 3 bis 1 : 10 : 10.

Es zeigte sich aber, daß in diesem Fall bei der Herstellung wasserunlöslicher Peroxycarbonsäuren mit mindestens 6 C-Atomen starke Schaumbildung auftritt, wenn die Ausgangscarbonsäure in der Reaktionsmischung nicht gelöst vorliegt sondern eine Suspension bildet.

Das Verfahren der EP-A-01 27 782 — veröffentlicht nach dem Prioritätstag der vorliegenden Erfindung — Lehrt, die Schaumbildung während der Umsetzung durch Zugabe eines Phosphanoxids zu mindern. Dieses Verfahren geht auch von wasserunlöslichen aliphatischen oder aromatischen Carbonsäuren aus, welche in einer Wasserstoffperoxid, Wasser und Schwefelsäure enthaltenden Oxidationsmischung in Suspension peroxidiert werden.

Aufgabe der Erfindung ist ein weiteres Verfahren zur Herstellung von Peroxycarbonsäuren mit mindestens 6 C-Atomen, bei dem die Schaumbildung unterdrückt wird. Gegenstand der Erfindung ist ein Verfahren zur Herstellung wasserunlöslicher Peroxycarbonsäuren durch Umsetzung aliphatischer Carbonsäuren mit 6 bis 16 C-Atomen beziehungsweise aromatischer Carbonsäuren mit 7 bis 9 C-Atomen mit einer Oxidationsmischung aus Wasserstoffperoxid, Wasser und Schwefelsäure, wobei die jeweilige Carbonsäure in der Oxidationsmischung eine Suspension bildet, dadurch gekennzeichnet, daß man die Umsetzung in Gegenwart von 0,01 bis 10 Gew.-%, bevorzugt 0,25 bis 1,6 Gew.-%.

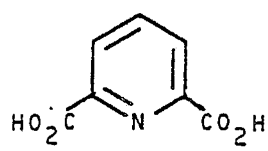

Pyridin-2.6.-dicarbonsäure (PDCA), bezogen auf das eingesetzte Wasserstoffperoxid, vornimmt.

Man führt die Umsetzung mit einem Molverhältnis von Wasserstoffperoxid zu Carbonsäure von 1 bis 10 zu 1, bevorzugt 1,5 bis 3 zu 1, aus.

Besonders geeignet sind aliphatische Mono- und Dicarbonsäuren mit einer Gesamtzahl von 9 bis 13 C-Atomen und darunter bevorzugt Dicarbonsäuren der Formel

$$HO-\overset{O}{\overset{\|}{C}}-(CH_2)_n-\overset{O}{\overset{\|}{C}}-OH \quad (n = 7 \text{ bis } 11),$$

insbesondere die Azelain-, die Dodecandi- und die Brassylsäure. Als aromatische Säuren bevorzugt man Phthalsäure und deren Isomere.

Bei der Durchführung des erfindungsgemäßen Verfahrens erweist es sich als günstig, Schwefelsäure und Carbonsäure im Molverhältnis 1 bis 10 zu 1, bevorzugt 2 bis 4 zu 1, einzusetzen. Für die Auswahl der Molverhältnisse allgemein ist wesentlich, daß die Ausgangscarbonsäure mit der jeweiligen Oxidationsmischung eine Suspension bildet.

Ferner zeigte sich, daß die besten Ergebnisse erhalten werden, wenn man das PDCA in einer Mischung aus wasserhaltigem Wasserstoffperoxid und Schwefelsäure löst und darauf die Carbonsäure in dieser Mischung suspendiert.

Die Umsetzung wird bei Temperaturen zwischen 40-70 °C, bevorzugt bei 45-60 °C, durchgeführt.

Wasserstoffperoxid wird in Konzentrationen von 30 bis 99 Gew.-%, bevorzugt 40 bis 50 Gew.-%, eingesetzt ; Schwefelsäure in Konzentrationen von 20 bis 98 Gew.-%, bevorzugt 90 bis 98 Gew.-%.

Die Verwendung von PDCA in der Oxidationsmischung ist nicht zu verwechseln mit dem bekannten Einsatz dieser Verbindung zur Stabilisierung von Peroxycarbonsäurezubereitungen (US-PS-3 956 159).

Durch das erfindungsgemäße Vorgehen wird die Herstellung von Peroxycarbonsäuren mit mindestens 6 C-Atomen aus einer suspendierten Carbonsäure enthaltenden Oxidationsmischung technisch machbar, da im Gegensatz zu früher die Schaumbildung so weit unterdrückt wird, daß man handhabbare, d. h. zum Beispiel pumpfähige Mischungen erhält.

Die ohne Zusatz von PDCA auftretende Schaumbildung aus feinsten Gasbläschen in der gesamten Reaktionsmischung schafft jedoch nicht nur verfahrenstechnische Probleme sondern beeinflußt auch die Reaktion, da das Benetzen der suspendierten Carbonsäure mit der Oxidationsmischung durch die anhängenden Gasbläschen behindert wird.

Das Reaktionsprodukt wird üblicherweise durch Filtrieren oder Zentrifugieren aus der Reaktionsmischung abgetrennt und getrocknet. Soll reine Peroxycarbonsäure ohne zusätzliche Phlegmatisierungssalze gewonnen werden, so muß das Produkt mineralsäurefrei gewaschen werden.

Die Peroxycarbonsäuren lassen sich hervorragend stabilisieren, wenn man vor, während oder nach der Umsetzung der Oxidationsmischung ein Phlegmatisierungsmittel zusetzt.

Als solche Phlegmatisierungsmittel kommen Alkali-, Magnesium-, Erdalakli- oder Erdmetallsulfate oder Borsäure in Frage.

Diese Stoffe können sowohl in fester Form oder als wässrige Lösungen bzw. Suspensionen zugesetzt werden. Es ist auch möglich, sie vor oder während der Umsetzung oder/und auf dem susperierten Produkt vor dessen Abtrennen aus der Reaktionslösung in situ zu erzeugen.

Besonders geeignet ist hierfür ein Verfahren, bei dem man die Umsetzung der wasserunlöslichen Carbonsäure mit der Oxidationsmischung in Anwesenheit von PDCA bei 45-60 °C vornimmt, darauf das Reaktionsgemisch auf Temperaturen von 45-5 °C, bevorzugt 40-32 °C, abkühlt, eine wässrige Alkalisulfatlösung zusetzt, den pH-Wert der Mischung auf 2-6 bevorzugt 3 bis 4 mit Hilfe von Alkalilaugen oder Alkalicarbonatlösungen einstellt und danach das Reaktionsprodukt in bekannter Weise aufarbeitet.

Als Alkalihydroxide oder Alkalicarbonate werden wässrige Lösungen mit 5-50 Gew.-%, bevorzugt 30-50 Gew.-% Alkalihydroxid, eingesetzt. Von den drei Alkalihydroxiden bzw. -carbonaten ist das Natriumderivat besonders bevorzugt.

Der obengenannte pH-Wert von 2-6 kann ebenfalls eingestellt werden nach Beendigung der Reaktion zur Herstellung der Peroxycarbonsäure durch Zugabe von entsprechenden Mengen an Magnesiumhydroxid oder-oxid, oder von Erdalkalihydroxiden, wie deren Carbonaten, oder von Erdmetallhydroxiden, wie deren Carbonaten, außerdem von Alkalialuminaten oder auch von Natriummetaborat. Auch diese Stoffe können in gelöster oder suspendierter Form, d. h. in wässrigem Medium, zugesetzt werden.

Das Phlegmatisierungsmittel soll bevorzugt in Mengen von 0,1 bis 80 Gew.-%, bezogen auf das fertige Produkt, anwesend sein.

Die Lagerstabilität wird durch das Phlegmatisierungsmittel in starker Weise erhöht.

Bei Verwendung von Natriumsulfat, das entweder fest oder in wässriger Lösung zugesetzt, oder in situ gebildet wird — auch können alle drei Möglichkeiten gleichzeitig verwendet werden — wird der Restfeuchtegehalt der Peroxycarbonsäuren nach dem Zentrifugieren um bis zu 50 % vermindert.

Besonders stark tritt dieser Effekt auf, wenn die in situ-Bildung von Natriumsulfat aus der Reaktionsgemisch vorhandenen Schwefelsäure und zugesetzter Natronlauge bei Temperaturen oberhalb des Umwandlungspunktes von Natriumsulfat-Dekahydrat zu wasserfreiem Natriumsulfat, d. h. bei Temperaturen ab 32,3-32,4 °C, vorgenommen wird.

Die Erfindung wird anhand der folgenden Beispiele erläutert.

## Vergleichsbeispiel

Entsprechend den Angaben aus Beispiel 1 wird eine Oxidationsmischung mit darin suspendierter Dodecandisäure (DPDDA) umgesetzt.

Nach kurzer Zeit schäumt die Reaktionsmischung in der Weise stark auf, daß sich nicht nur Schaum an der Flüssigkeitsoberfläche bildet sondern auch die Mischung selbst so sehr von feien Gasblasen durchsetzt ist, daß sie eine fast als sahneartig zu bezeichnende Konsistenz annimmt. In dieser Form ist eine Handhabung der nicht mehr pumpfähigen Mischung im technischen Maßstab nicht denkbar.

Verfährt man dagegen gemäß den nachfolgenden Beispielen 1-8, wird die Schaumbildung so weitgehend unterdrückt, daß man problemlos in größerem Maßstab arbeiten kann.

Auch die an die Umsetzung sich anschließende teilweise Neutralisation der Schwefelsäure läßt sich durch die jetzt ermöglichte leichte Verteilbarkeit des Neutralisierungsmittels ohne jede Gefahr der Zersetzung von Aktivsauerstoff durch lokale Ansammlung von Alkali durchführen. Ferner ist die schließlich erhaltene Suspension aus Persäure und Phlegmatisierungsmittel fließfähig und ohne weiteres zentrifugierbar.

## Beispiel 1

In eine Oxidationsmischung, bestehend aus 170 g Wasserstoffperoxid (50 gew.-%ig) und 204 g Schwefelsäure (96 gew.-%ig), sowie 1,3 g Pyridin-2.6-dicarbonsäure, werden 115 g Dodecandisäure

eindosiert und 8 h unter Rühren auf 50 °C erhitzt. Nach dem Abkühlen auf 8 °C wird der Ansatz bei dieser Temperatur mit 300 g Natriumsulfatlösung (13 gew.-%ig) versetzt und anschließend mit 517 g Natriumhydroxidlösung (30 gew.-%ig) bis zum Erreichen des pH-Wertes von 3,5 neutralisiert. Anschließend wird zentrifugiert und getrocknet. Trocknung bei 40 °C.

Die Ausbeute an Persäure beträgt : 106,2 g ≙ 81 % d. Th.
An Gesamt-AO-Gehalt wird gefunden : 3,75 %.
Die Dodecandisäurebilanz beträgt : 89,9 %.
Gehalt und Lagerstabilität :

| | 0 Wochen | 4 Wochen | 8 Wochen | 12 Wochen |
|---|---|---|---|---|
| DPDDA-Gehalt | 30,7 | 30,7 | 30,5 | 30,5 |
| AO | 3,75 | 3,75 | 3,73 | 3,73 |

In gleicher Weise ohne PDCA-Zusatz hergestellte DPDDA verliert in 12 Wochen ca. 10 % ihres AO-Gehaltes.

## Beispiel 2

In einer Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig), sowie 0,14 g Pyridin-2.6.-dicarbonsäure, werden 115 g Dodecandisäure suspendiert und 8 h unter Rühren auf 60 °C erhitzt. Nach dem Abkühlen auf 8 °C wird der Ansatz bei dieser Temperatur mit 500 g Natriumsulfatlösung (13 gew.-%ig) versetzt und anschließend mit 526 g Natriumhydroxidlösung (30 gew.-%ig) bis zum Erreichen des pH-Wertes von 3,5 neutralisiert. Anschließend wird zentrifugiert und getrocknet. Trocknung bei 40 °C.

Die Ausbeute an Persäure beträgt : 115 g ≙ 87,7 % d. Th.
An Gesamt-AO-Gehalt wird gefunden : 3,79 %.
Die Dodecandisäurebilanz beträgt : 95,8 %.
Der Gehalt an DPDDA beträgt : 31,0 %.

## Beispiel 3

In einer Oxidationsmischung, bestehend aus 255 g Wasserstoffperoxid (50 gew.-%ig), 510 g Schwefelsäure (96 gew.-%ig) und 64 g Natriumsulfatlösung (13 gew.-%ig), sowie 0,4 g Pyridin-2.6.-dicarbonsäure, werden 287,5 g Dodecandisäure suspendiert und 8 h unter Rühren auf 60 °C erhitzt. Nach dem Abkühlen auf 20 °C wird der Ansatz bei dieser Temperatur mit 1l destilliertem Wasser versetzt und abgesaugt. Der Rückstand wird mit kaltem, destilliertem Wasser gewaschen und getrocknet.

Die Ausbeute an Persäure beträgt : 303,9 g ≙ 92,8 % d. Th.
An Gesamt-AO-Gehalt wird gefunden : 11,64 %
Die Dodecandisäurebilanz beträgt : 97,0 %
Der Gehalt an DPDDA beträgt : 95,4 %.

## Beispiel 4

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,16 g Pyridin-2.6.-dicarbonsäure, werden 115 g Dodecandisäure eindosiert und 6 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschließend mit 521 g Natriumhydroxidlösung (30 gew.-%ig) bis zum Erreichen des pH-Wertes von 3,5 neutralisiert und mit 160 g Natriumsulfat konditioniert. Anschließend wird separiert und bei 40 °C getrocknet.

Die Ausbeute an Persäure beträgt : 120,1 g ≙ 91,5 % d. Th.
An Gesamt-AO-Gehalt wird gefunden : 4,23 %
Die Dodecandisäurebilanz beträgt : 99,1 %
Der Gehalt an DPDDA beträgt : 34,6 %

## Beispiel 5

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,8 g Pyridin-2.6.-dicarbonsäure werden 115 g Dodecandisäure eindosiert und 6 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschließend mit 521 g Natriumhydroxidlösung (30 gew.-%ig) bis zum Erreichen des

pH-Wertes von 3,5 neutralisiert und mit 160 g Natriumsulfat konditioniert. Anschließend wird separiert und getrocknet.

Die Ausbeute an Persäure beträgt : 118,1 g ≙ 90,1 % d. Th.
An Gesamt-AO-Gehalt wird gefunden : 4,49 %.
Die Dodecandisäurebilanz beträgt : 95,6 %.
Der Gehalt an DPDDA beträgt : 36,8 %.

Beispiel 6

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,16 g Pyridin-2.6.-dicarbonsäure, werden 115 g Dodecandisäure eindosiert und 4 h unter Rühren auf 60 °C erhitzt, Nach dem Abkühlen auf 40 °C wird der Ansatz bei dieser Temperatur mit 526 g Natriumsulfatlösung (30 gew.-%ig) versetzt und anschließend mit 521 g Natriumhydroxidlösung (30 gew.-%ig) bis zum Erreichen des pH-Wertes von 3,5 neutralisiert und mit 160 g festem Natriumsulfat konditioniert. Anschließend wird zentrifugiert und getrocknet.

Die Ausbeute an Persäure beträgt : 120,2 g ≙ 91,7 % d. Th.
An Gesamt-AO-Gehalt wird gefunden : 4,54 %
Die Dodecandisäurebilanz beträgt : 99,3 %
Der Gehalt an DPDDA beträgt : 37,1 %.

Beispiel 7

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,8 g Pyridin-2.6.-dicarbonsäure, werden 115 g Dodecandisäure eindosiert und 4 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschließend mit 523 g Natriumhydroxidlösung (30 gew.-%ig) bis zum Erreichen des pH-Wertes von 3,5 neutralisiert und mit 160 g Natriumsulfat konditioniert. Anschließend wird separiert und getrocknet.

Die Ausbeute an Persäure beträgt : 119,5 g ≙ 91,2 % d. Th.
An Gesamt-AO-Gehalt wird gefunden : 4,42 %.
Die Dodecandisäurebilanz beträgt : 98,5 %.
Der Gehalt an DPDDA beträgt 36,2 %.

Beispiel 8

In eine Oxidationsmischung, bestehend aus 127 g Wasserstoffperoxid (40 gew.-%ig) und 204 g Schwefelsäure (96 gew.-%ig), sowie 0,16 g Pyridin-2.6.-dicarbonsäure, werden 115 g Dodecandisäure eindosiert und 4 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschließend mit 523 g Natriumhydroxidlösung (30 gew.-%ig) bis zum Erreichen des pH-Wertes von 3,5 neutralisiert und mit 160 g festem Natriumsulfat konditioniert. Anschließend wird zentrifugiert und getrocknet. Trocknung bei 40 °C.

Die Ausbeute an Persäure beträgt : 115,2 g ≙ 87,8 % d. Th.
An Gesamt-AO-Gehalt wird gefunden : 4,24 %.
Die Dodecandisäurebilanz beträgt : 95,3 %.
Der Gehalt an DPD beträgt : 34,7 %.

Beispiel 9

In einer Oxidationsmischung, bestehend aus 120 g Wasserstoffperoxid (85 gew.-%ig), 306 g Schwefelsäure (96 gew.-%ig), sowie 0,16 g Pyridin-2.6-dicarbonsäure, werden 99,7 g Isophthalsäure suspendiert und 24 h unter Rühren auf 50 °C erhitzt. Nach dem Abkühlen auf 8 °C wird der Ansatz bei dieser Temperatur mit 300 ml Wasser versetzt und abgesaugt. Der Rückstand wird mit kaltem, destilliertem Wasser gewaschen und getrocknet.

Die Ausbeute an Persäure beträgt : 91,9 g ≙ 77,3 % d. Th.
An Gesamt-AO-Gehalt wird gefunden : 14,37 %.
Die Isophthalsäurebilanz beträgt : 89,4 %.
Der Gehalt an DPIPA beträgt : 88,4 %.

Beispiel 10

In einer Oxidationsmischung, bestehend aus 34 g Wasserstoffperoxid (50 gew.-%ig), 66 g Schwefelsäure sowie 0.048 g Pyridin-2.6-dicarbonsäure, werden 36.6 g Brassylsäure suspendiert und 6 h unter

Rühren auf 60 °C erhitzt. Nach dem Abkühlen auf 15 °C wird der Ansatz bei dieser Temperatur mit 250 ml Wasser versetzt und abgesaugt. Der Rückstand wird mit kaltem destilliertem Wasser gewaschen und getrocknet.

Die Ausbeute an Persäure beträgt : 37,9 g ≙ 92,0 % d. Th.
An Gesamt-AO-Gehalt wird gefunden : 11,0 %.
Die Brassylsäurebilanz beträgt : 95,9 %.
Der Gehalt an DPBA beträgt : 95,3 %.

Beispiel 11

In einer Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig), sowie 0,16 g Pyridin-2.6-dicarbonsäure, werden 94,1 g Azelainsäure (techn.) suspenddiert und 4 h unter Rühren auf 60 °C erhitzt. Nach dem Abkühlen auf 20 °C wird der Ansatz bei dieser Temperatur mit 500 ml Wasser versetzt und abgesaugt. Der Rückstand wird mit kaltem, destilliertem Wasser gewaschen und getrocknet.

Die Ausbeute an Persäure beträgt : 91,1 g.
An Gesamt-AO-Gehalt wird gefunden : 13,90 %.
Der Gehalt an DPAA beträgt : 94,9 %.

Beispiel 12

In eine Oxidationsmischung, bestehend aus 20.4 g Wasserstoffperoxid (50 gew.-%ig), 30.6 g Schwefelsäure (96 gew.-%ig), sowie 0.1 g Pyridin-2.6-dicarbonsäure, werden 34 g Decansäure eindosiert und 3 h unter Rühren auf 35 °C erhitzt. Nach dem langsamen Abkühlen auf 20 °C wird der Ansatz mit 50 ml Wasser versetzt und abgesaugt. Der Rückstand wird mit kaltem, destilliertem Wasser gewaschen und getrocknet.

Die Ausbeute an Persäure beträgt : 33,9 g ≙ 90,2 % d. Th.
An Gesamt-AO-Gehalt wird gefunden : 7,85 %.
Die Decansäurebilanz beträgt : 99,9 %.
Der Gehalt an PDA beträgt : 91,2 %.

**Patentansprüche**

1. Verfahren zur Herstellung wasserunlöslicher Peroxycarbonsäuren durch Umsetzung von aliphatischen Carbonsäuren mit 6 bis 16 C-Atomen oder aromatischen Carbonsäuren mit 7 bis 9 C-Atomen mit einer Oxidationsmischung aus Wasserstoffperoxid, Wasser und Schwefelsäure, wobei die jeweilige Carbonsäure in der Oxidationsmischung eine Suspension bildet, dadurch gekennzeichnet, daß man die Umsetzung in Gegenwart von 0,01 bis 10 Gew.-%, bevorzugt 0,25 bis 1,6 Gew.-%.

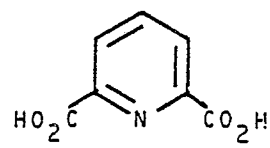

bezogen auf das eingesetzte Wasserstoffperoxid, vornimmt.

2. Verfahren nach Anspruch 1, dadurch gekennzeichnet, daß man die Umsetzung mit einem Molverhältnis von Wasserstoffperoxid zu Carbonsäure von 1 bis 10 zu 1, bevorzugt 1,5 bis 3 zu 1, vornimmt.

3. Verfahren nach den Ansprüchen 1 bis 2, dadurch gekennzeichnet, daß man die Pyridin-2.6-dicarbonsäure der Mischung aus Wasserstoffperoxid und Schwefelsäure zusetzt und dann die Umsetzung mit Carbonsäure durchführt.

4. Verfahren nach den Ansprüchen 1 bis 3, dadurch gekennzeichnet, daß man Schwefelsäure und Carbonsäure im Molverhältnis von 1 bis 10 zu 1, bevorzugt 2 bis 4 zu 1, einsetzt.

5. Verfahren nach den Ansprüchen 1 bis 4, dadurch gekennzeichnet, daß man als Phlegmatisierungsmittel eine wässrige Alkali-, Magnesium-, Erdalkali- oder Erdmetallsulfatlösung, bevorzugt eine Natriumsulfatlösung, vor, während oder nach der Umsetzung zusetzt.

6. Verfahren nach den Ansprüchen 1 bis 4, dadurch gekennzeichnet, daß man als Phlegmatisierungsmittel Alkalialuminat, bevorzugt Natriumaluminat, in wässriger Lösung vor, während oder nach der Umsetzung zusetzt.

7. Verfahren nach den Ansprüchen 1 bis 4, dadurch gekennzeichnet, daß man als Phlegmatisierungsmittel eine wässrige Lösung von Natriummetaborat während oder nach der Umsetzung zugibt.

8. Verfahren nach den Ansprüchen 1 bis 5, dadurch gekennzeichnet, daß man die Umsetzung bei Temperaturen von 45 bis 60 °C vornimmt, darauf das Reaktionsgemisch auf Temperaturen von 45-5 °C,

bevorzugt auf 40-32 °C, abkühlt und eine wässrige Alkalisulfatlösung zusetzt, worauf man den pH-Wert der Reaktionsmischung auf 2 bis 6 mit Alkalilaugen oder Alkalicarbonatlösungen einstellt und das Reaktionsprodukt in bekannter Weise aufarbeitet.

9. Verfahren nach Anspruch 1 bis 8, dadurch gekennzeichnet, daß man als Carbonsäure aliphatische Mono- oder Dicarbonsäuren, bevorzugt Azelainsäure, Dodecandisäure oder Brassylsäure, einsetzt.

**Claims**

1. A process for the production of water-insoluble peroxycarboxylic acids by reaction of aliphatic $C_6$-$C_{16}$ carboxylic acids or aromatic $C_7$-$C_9$ carboxylic acids with an oxidation mixture of hydrogen peroxide, water and sulfuric acid, the particular carboxylic acid forming a suspension in the oxidation mixture, characterized in that the reaction is carried out in the presence of 0.01 to 10 % by weight and preferably 0.25 to 1.6 % by weight of

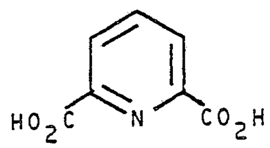

based on the introduced hydrogen peroxide.

2. A process as claimed in claim 1, characterized in that the reaction is carried out with a molar ratio of hydrogen peroxide to carboxylic acid of 1 to 10 : 1 and preferably 1.5 to 3 : 1.

3. A process as claimed in claims 1 and 2, characterized in that the pyridine-2,6-dicarboxylic acid is added to the mixture of hydrogen peroxide and sulfuric acid and the reaction with carboxylic acid subsequently carried out.

4. A process as claimed in claims 1 to 3, characterized in that sulfuric acid and carboxylic acid are used in a molar ratio of 1 to 10 : 1 and preferably 2 to 4 : 1.

5. A process as claimed in claims 1 to 4, characterized in that an aqueous alkali, magnesium, alkaline earth or earth metal sulfate solution, preferably a sodium sulfate solution, is added as desensitizing agent before, during or after the reaction.

6. A process as claimed in claims 1 to 4, characterized in that alkali aluminate, preferably sodium aluminate, in aqueous solution is added as desensitizing agent before, during or after the reaction.

7. A process as claimed in claims 1 to 4, characterized in that aqueous solution of sodium metaborate is added as desensitizing agent during or after the reaction.

8. A process as claimed in claims 1 to 5, characterized in that the reaction is carried out at temperatures of 45 to 60 °C, the reaction mixture is then cooled to temperatures of 45 to 5 °C and preferably to temperatures of 40 to 32 °C and an aqueous alkali sulfate solution is added, after which the pH value of the reaction mixture is adjusted to 2-6 with alkali hydroxides or alkali carbonate solutions and the reaction product is worked up in known manner.

9. A process as claimed in claims 1 to 8, characterized in that aliphatic mono- or dicarboxylic acids, preferably azelaic acid, dodecanedioic acid or brassylic acid, are used as the carboxylic acid.

**Revendications**

1. Procédé d'obtention d'acides peroxycarboxyliques insolubles dans l'eau par réaction d'acides carboxyliques aliphatiques ayant de 6 à 16 atomes de carbone ou d'acides carboxyliques aromatiques ayant de 7 à 9 atomes de carbone avec un mélange d'oxydation à base de peroxyde d'hydrogène, d'eau et d'acide sulfurique, dans lequel l'acide carboxylique respectif forme une suspension dans le mélange d'oxydation, caractérisé en ce que la réaction est effectuée en présence de 0,01 à 10 % en poids et de préférence de 0,25 à 1,6 % en poids de

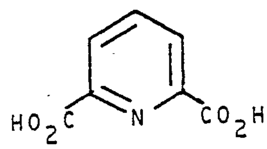

calculé sur la quantité de peroxyde d'hydrogène introduite.

2. Procédé selon la revendication 1, caractérisé en ce que la réaction est effectuée avec un rapport molaire de peroxyde d'hydrogène par rapport à l'acide carboxylique de 1 à 10 pour 1, de préférence de 1,5 à 3 pour 1.

3. Procédé selon les revendications 1 à 2, caractérisé en ce que l'on ajoute l'acide pyridyl-2,6-dicarboxylique au mélange à base de peroxyde d'hydrogène et d'acide sulfurique et qu'ensuite on effectue la réaction avec l'acide carboxylique.

4. Procédé selon les revendications 1 à 3, caractérisé en ce que l'on met en jeu l'acide sulfurique et l'acide carboxylique dans un rapport molaire de 1 à 10 pour 1, de préférence de 2 à 4 pour 1.

5. Procédé selon les revendications 1 à 4, caractérisé en ce que l'on ajoute en tant qu'agent de phlegmatisation (relargage) une solution aqueuse d'un sulfate de métal alcalin, de magnésium, de métal alcalino-terreux ou de métal terreux, de préférence une solution de sulfate de sodium, avant, pendant ou après la réaction.

6. Procédé selon les revendications 1 à 4, caractérisé en ce que comme agent de phlegmatisation (relargage) on ajoute un aluminate alcalin, de préférence l'aluminate de sodium en solution aqueuse, avant, pendant ou après la réaction.

7. Procédé selon les revendications 1 à 4, caractérisé en ce que l'on ajoute comme agent de phlegmatisation (relargage) une solution aqueuse de métaborate de sodium pendant ou après la réaction.

8. Procédé selon les revendications 1 à 5, caractérisé en ce que l'on effectue la réaction à des températures allant de 45 à 60 °C, par la suite refroidit le mélange réactionnel à des températures de 45 à 5 °C de préférence de 40 à 32 °C, et ajoute une solution aqueuse de sulfate alcalin, après quoi on ajuste la valeur du pH du mélange réactionnel de 2 à 6 avec une lessive alcaline ou une solution de carbonate alcalin et on transforme le produit de la réaction d'une façon connue.

9. Procédé selon les revendications 1 à 8, caractérisé en ce que l'on met en œuvre comme acide carboxylique, des acides aliphatiques mono- ou dicarboxylique de préférence l'acide Azelaïque, l'acide dodecyldicarboxylique ou l'acide brassidique ;